# EUROPEAN PATENT SPECIFICATION

(11) **EP 1 995 801 B1**
(45) Date of publication and mention of the grant of the patent: **13.06.2012**
(21) Application number: 07714353.5
(22) Date of filing: 16.02.2007
(51) Int. Cl.: H01L 51/30

(54) **ORGANIC SEMICONDUCTOR DEVICE AND ORGANIC SEMICONDUCTOR THIN FILM**
ORGANISCHES HALBLEITERELEMENT UND ORGANISCHER HALBLEITERDÜNNFILM
ÉLÉMENT ET FILM MINCE SEMI-CONDUCTEURS ET ORGANIQUES

(30) Priority: 10.03.2006 JP 2006066166
(43) Date of publication of application: 26.11.2008
(73) Proprietor: Sony Corporation, Tokyo 108-0075 (JP)
(72) Inventor: KATSUHARA, Mao, Tokyo 108-0075 (JP); UGAWA, Akito, Tokyo 108-0075 (JP); MIYAMOTO, Yoshihiro, Tokyo 108-0075 (JP); KUNIKIYO, Toshiyuki, Tokyo 108-0075 (JP)
(74) Representative: Hart-Davis, Jason
(86) International application number: PCT/JP2007/052823
(87) International publication number: WO 2007/105408

(56) References cited:
- DE-U1-202005 009 955
- JP-A- 2002 308 856
- JP-A- 2006 206 503
- DARABI H R ET AL: "Synthesis, Properties and Molecular Structure of a New Isomer of [2.4]-Paracyclophanetetraene: (E,Z,E,Z)-[2.4]-Paracyclophanetetraene", TETRAHEDRON LETTERS, ELSEVIER, AMSTERDAM, NL, vol. 36, no. 52, 25 December 1995 (1995-12-25), pages 9525-9526, XP004026675, ISSN: 0040-4039, DOI: DOI:10.1016/0040-4039(95)02065-9
- ZHONGYING HU, JERRY L. ATWOOD, MICHAEL P. CAVA: "A Simple Route to Sulfur Bridged Annulenes", JOURNAL OF ORGANIC CHEMISTRY, vol. 59, December 1994 (1994-12), pages 8071-8075, XP002610846,
- HOSSEIN REZA DARABI ET AL.: 'Synthesis, Properties and Molecular Structure of a New Isomer of [2.4]-Paracyclophanetetraene: (E,Z,E,Z)-[2.4]-Paracyclophanetetraene' TETRAHEDRON LETTERS vol. 36, no. 52, 1995, pages 9525 - 9526, XP004026675

## Description

### Technical Field

The present invention relates to an organic semiconductor thin film and an organic semiconductor device including the organic semiconductor thin film.

### Background Art

Field effect transistors (FET) including thin film transistors (TFT) currently used for many electron apparatuses each include, for example, a channel forming region and source/drain regions (source/drain electrodes) formed on a silicon semiconductor substrate or a silicon semiconductor layer, a gate insulating layer composed of SiO₂ and formed on the surface of the silicon semiconductor substrate or the silicon semiconductor layer, and a gate electrode provided opposite to the channel forming region with the gate insulating layer formed therebetween. Alternatively, field effect transistors each include a gate electrode formed on a support, a gate insulating layer formed on the support including the gate electrode, and a channel forming region and source/drain regions (source/drain electrodes) formed on the gate insulating layer. Field effect transistors having such structures are manufactured using an expensive semiconductor manufacturing apparatus. Therefore, there is strong demand for decreasing the manufacturing cost.

Therefore, in recent years, researches on so-called organic semiconductor devices have been intensively advanced. For example, it is known to be possible to manufacture FET having a mobility of over 1 cm²·V⁻¹·sec⁻¹ in a channel forming region formed by depositing a thin film of pentacene, which is a polyacene compound and an organic semiconductor material, by an evaporation process. Therefore, it is greatly expected that FET exhibiting excellent characteristics can be manufactured using pentacene.

However, a polyacene compound is a compound in which benzene rings are linearly connected, and a polyacene compound having no substituent has the property that the solubility in organic solvents decreases as the number of benzene rings increases. In particular, in a polyacene compound higher than pentacene in which five benzene rings are connected, solubility in almost solvents is lost, and it is very difficult to form a uniform film on the basis of a spin coating method. Even if possible, the organic solvent and temperature condition are very limited (for example, trichlorobenzene, 60°C to 180°C). Also it is widely known that stability decreases as the number of benzene rings increases, and pentacene is oxidized with atmospheric oxygen. Namely, pentacene has low oxidation resistance.

As an example of polyacene compounds having substituents, 2,3,9,10-tetramethylpencetane has been reported (refer to Wudl and Bao, Adv. Mater Vol. 15, No 3 (1090-1093), 2003). However, 2,3,9,10-tetramethylpencetane is only slightly dissolved in hot o-dichlorobenzene and actually used for forming a channel forming region constituting TFT by a vacuum evaporation process.

Also Japanese Unexamined Patent Application Publication No. 2004-256532 discloses that 2,3,9,10-tetramethylpentacene and 2,3-dimethylpentacene are dissolved in o-dichlorobenzene. However, such compounds are dissolved at 120°C, and it is not described that the compounds are actually dissolved at room temperature.
DE 20 2005 009 955 U1 describes an electrically driven element for producing light emission of varying colours. The element contains an active layer, which may contain oligophenylenevinylenes. Darabi et al., Tetrahedron Lett., 36:52 (1995), 9525-9526, disclose (E,Z,E,Z)-[2.4]paracyclophanetetraene. Hu et al., J. Org. Chem., 59 (1994), 8071-8075 describes syntheses of sulfur bridged annulenes.

### Disclosure of Invention

As described above, polyacene compounds are expected to have an excellent function as an organic semiconductor material, but are difficult to dissolve in organic solvents at low temperatures (e.g., room temperature) and unsuitable for use in methods not using vacuum technology, such as a spin coating method, a printing method, and a spray method.

Accordingly, an object of the present invention is to provide an organic semiconductor thin film composed of an organic semiconductor material which can be dissolved in an organic solvent at a low temperature (e.g., room temperature) and suitable for use in a coating process, and an organic semiconductor device including the organic semiconductor thin film based on the organic semiconductor material.

In order to achieve the object, in accordance with a first or second embodiment of the present invention, an organic semiconductor device includes a channel forming region including an organic semiconductor thin film which is composed of an organic semiconductor material having the following general formula (1) (wherein a hydrogen atom constituting a benzene ring may be substituted, and n is 0 or a positive integer).

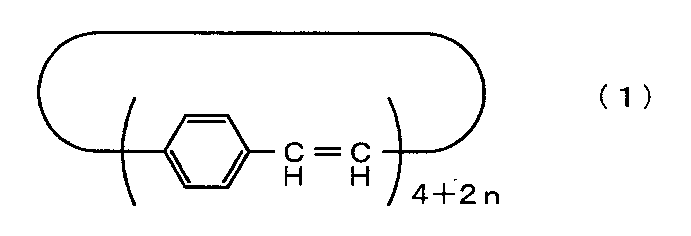

In order to achieve the object, in accordance with a first or third embodiment of the present invention, an organic semiconductor device includes a channel forming region including an organic semiconductor thin film which is composed of an organic semiconductor material having the following general formula (2) (wherein a hydrogen atom constituting a thiophene ring may be substituted, and n is 0 or a positive integer).

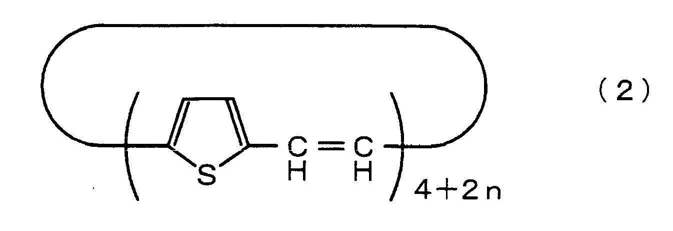

In order to achieve the object, in accordance with the first embodiment of the present invention, an organic semiconductor thin film is composed of an organic semiconductor material having the above general formula (1) (wherein a hydrogen atom constituting a benzene ring may be substituted, and n is 0 or a positive integer).

In order to achieve the object, in accordance with the second embodiment of the present invention, an organic semiconductor thin film is composed of an organic semiconductor material having the above general formula (2). (wherein a hydrogen atom constituting a thiophene ring may be substituted, and n is 0 or a positive integer).

In the organic semiconductor device according to the second or third embodiment of the present invention or in the organic semiconductor thin film according to the first or second embodiment of the present invention, a substituent may be an alkyl group (CₘH₂ₘ₊₁- wherein m = 1, 2, 3 ... ) or a halogen atom (specifically, a F atom, a Cl atom, a Br atom, or an I atom). In this case, when a hydrogen atom of a conjugated ring of the above-described material is substituted by any one of various substituents, the ionization potential, solubility, and steric hindrance of the molecule can be controlled. Substituents may be introduced into all or some of the hydrogen atoms constituting the benzene ring or the thiophene ring.

In the organic semiconductor device according to the second or third embodiment of the present invention or in the organic semiconductor thin film according to the first or second embodiment of the present invention, a tetramer is formed when n = 0, a hexamer is formed when n = 1, an octomer is formed when n = 2, and a decamer is formed when n = 3. These may be generally named "analogues".

The organic semiconductor material of the present invention can be dissolved in a wide variety of organic solvents at room temperature. Specifically, the organic semiconductor material is dissolved, at room temperature, in an amount required for a coating process such as a spin coating process, a dipping (dip coating) process, an air doctor coating process, a blade coating process, a rod coating process, a knife coating process, a squeeze coating process, a reverse roll coating process, a transfer roll coating process, a gravure coating process, a kiss coating process, a cast coating process, a spray coating process, a slit orifice coating process, a calender coating process, or a die coating process; a printing process such as a screen printing process, an ink jet printing process, an offset printing process, or a gravure printing process; or an application process such as a casting process or a spray process in a wide variety of organic solvents such as hydrocarbon solvents (e.g., hexane, heptane, octane, and cyclohexane), ester solvents (e.g., ethyl acetate and butyrolactone), alcohol solvents (e.g., octanol, hexanol, and benzyl alcohol), aromatic solvents (e.g., toluene, mesitylene, and benzene), ether solvents (e.g., diethyl ether and tetrahydrofuran), halogenated solvents (e.g., chloroform and dichloromethane), and ketone solvents (e.g., acetone and cyclopentanone).

The organic semiconductor device of the present invention may include source/drain electrodes, a channel forming region held between the source/drain electrode and the source/drain electrode, a gate insulating layer, and a gate electrode provided opposite to the channel forming region with the gate insulating layer provided therebetween, the channel forming region including an organic semiconductor thin film. Namely, the organic semiconductor device may be an organic field effect transistor (organic FET).

Examples of the structure of the organic field effect transistor include the four types of structures below. The organic semiconductor thin films constituting the respective organic semiconductor devices according to the first to third embodiments of the present invention or the organic semiconductor thin films according to the first and second embodiments of the present invention may be generally simply named "the organic semiconductor thin film of the present invention".

An organic field effect transistor having a first structure is a so-called bottom gate/bottom contact type organic field effect transistor including:
(A) a gate electrode formed on a substrate;
(B) a gate insulating layer formed on the gate electrode and the substrate;
(C) source/drain electrodes formed on the gate insulating layer; and
(D) a channel forming region formed between the source/drain electrodes on the gate insulating layer and including the organic semiconductor thin film of the present invention.

An organic field effect transistor having a second structure is a so-called bottom gate/top contact type organic field effect transistor including:
(A) a gate electrode formed on a substrate;
(B) a gate insulating layer formed on the gate electrode and the substrate;
(C) a channel forming region formed on the gate insulating layer and including the organic semiconductor thin film of the present invention; and
(D) source/drain electrodes formed on the organic semiconductor thin film.

An organic field effect transistor having a third structure is a so-called top gate/top contact type organic field effect transistor including:
(A) a channel forming region formed on a substrate and including the organic semiconductor thin film of the present invention;
(B) source/drain electrodes formed on the organic semiconductor thin film;
(C) a gate insulating layer formed on the source/drain electrodes and the organic semiconductor thin film; and
(D) a gate electrode formed on the gate insulating layer.

An organic field effect transistor having a fourth structure is a so-called top gate/bottom contact type organic field effect transistor including:
(A) source/drain electrodes formed on a substrate;
(B) a channel forming region formed on the source/drain electrodes and the substrate and including the organic semiconductor thin film of the present invention;
(C) a gate insulating layer formed on the organic semiconductor thin film; and
(D) a gate electrode formed on the gate insulating layer.

Examples of a material for forming the gate insulating layer include inorganic insulating materials such as silicon oxide-based materials, silicon nitride (SiN_{y}), Al₂O₃, and metal oxide high-dielectric insulating films; and organic insulating materials such as poly(methyl methacrylate) (PUMA), polyvinylphenol (PVP), poly(ethylene terephthalate) (PET), polyoxyethylene (POM), poly(vinyl chloride), poly(vinylidene fluoride), polysulfone, polycarbonate (PC), polyvinyl alcohol (PVA), and polyimide. These materials may be used in combination. Examples of the silicon oxide-based materials include silicon dioxide (SiO₂), BPSG, PSG, BSG, AsSG, PbSG, silicon oxynitride (SiON), SOG (spin-on-glass), and low-dielectric-constant SiOₓ materials (e.g., polyarylether, cycloperfluorocarbon polymer, benzocyclobutene, cyclic fluorocarbon resins, polytetrafluoroethylene, arylether fluoride, poly(imide fluoride), amorphous carbon, and organic SOG).

Examples of a method for forming the gate insulating layer include various printing methods such as a screen printing method, an ink-jet printing method, an offset printing method, and a gravure printing method; various coating methods such as an air doctor coating method, a blade coating method, a rod coating method, a knife coating method, a squeeze coating method, a reverse roll coating method, a transfer roll coating method, a gravure coating method, a kis coating method, a cast coating method, a spray coating method, a slit orifice coating method, a calender coating method, and a die coating method; a dipping method; a casting method; a spin coating method; a spray method; various CVD methods; and various PVD methods. Examples of the PVD methods include (a) various kinds of vacuum deposition methods such as an electron beam heating method, a resistance heating method, and a flash vapor deposition method; (b) a plasma deposition method; (c) various kinds of sputtering methods such as a bipolar sputtering method, a DC sputtering method, a DC magnetron sputtering method, a high-frequency sputtering method, a magnetron sputtering method, an ion beam sputtering method, and a bias sputtering method; and (d) a DC (direct current) method, an RF method, a multi-cathode method, an activated reactive method, a field deposition method, and various kinds of ion plating methods such as a high-frequency ion plating method and a reactive ion plating method.

Alternatively, the gate insulating layer can be formed by oxidizing or nitriding the surface of the gate electrode or by forming an oxide film or a nitride film on the surface of the gate electrode. As a method of oxidizing the surface of the gate electrode, there can be excemplified a thermal oxidation method, an oxidation method using O₂ plasma, and an anodization method depending on the material constructing the gate electrode. As a method of nitriding the surface of the gate electrode, there can be exemplified a nitriding method using N₂ plasma depending on the material constructing the gate electrode. Alternatively, when the gate electrode is made of gold (Au), it is possible to form the gate insulating layer on the surface of the gate electrode by coating the surface of the gate electrode in a self-organization monner using a method such as a dipping method on the basis of insulating molecules having functional groups capable of chemically forming bonds with the gate electrode like a straight hydrocarbon of which one end is modified by a mercapto group.

Further, as the materials constructing the gate electrode, the source/drain electrodes and various kinds of wirings, there can be exemplified metals such as platinum (Pt), gold (Au), palladium (Pd), chromium (Cr), nickel (Ni), molybdenum (Mo), niobium (Nb), neodymium (Nd), aluminum (Al), silver (Ag), tantalum (Ta), tungsten (W), copper (Cu), rubidium (Rb), rhodium (Rh), titanium (Ti), indium (In), and tin (Sn), alloys containing these metal elements, conductive particles made of these metals, conductive particles of alloys containing these metals, polysilicon, amorphous silicon, tin oxide, indium oxide, and indium tin oxide (ITO), and a laminated structure of layers containing these elements. Further, as the materials constructing the gate electrode, the source/drain electrodes, and various kinds of wirings, there can be exmplified organic conductive materials such as poly(3,4-ethylenedioxythiophene)/poly(styrenesulfonic acid) (PEDOT/PSS).

As methods of forming the source/drain electrodes, the gate electrode, and various kinds of wiring, depending on the materials constructing the source/drain electrodes, the gate electrode, and various kinds of wirings, there can be used any one of a spin coating method; the above-mentioned various kinds of printing methods using various conductive pastes or various conductive polymer solutions; the above-mentioned various kinds of coating methods; a lift-off method; a shadow mask method; an electrolytic plating method, an electroless plating method, and a plating method of a combination of the electrolytic plating and the electroless plating; a spray method; the above-mentioned various kinds of PVD methods; and various kinds of CVD methods including an MOCVD method, and combinations of the above-mentioned methods and patterning techniques if necessary.

As the substrate, there can be exemplified various kinds of glass substrates, various kinds of glass substrates with insulating films formed on their surfaces, a quartz substrate, a quartz substrate with an insulating film formed on its surface, and a silicon substrate with an insulating film formed on its surface. Further, as the substrate, there can be exemplified plastic film or plastic sheet plastic substrates consisting of polymer materials such as polyethersulfone (PES), polyimide, polycarbonate (PC), poly(ethylene terephthalate) (PET), poly (methyl methacrylate) (PMMA), poly (vinyl alcohol) (PVA), and poly(vinyl phenol) (PVP). If a substrate composed of a polymer material with flexibility is used, then an organic semiconductor device can be assembled into or unified with display devies and electronic devices having curved-surface shapes. In addition, conductive substrates (substrates made of metals such as gold and graphite with high orientation) can be used as the substrate. Also, it is frequently observed that an organic semiconductor device is provided on a supporting member depending on the arrangement and structure of the organic semiconductor device. The supporting member in such a case also can be constructed using the above-mentioned material.

When the organic semiconductor device is applied to and used with display devices and various kinds of electronic devices, the organic semiconductor device may be formed as a monolithic integrated circuit in which a large number of organic semiconductor devices are integrated on the substrate. Each organic semiconductor device can be cut and separately used as a discrete component. Also, the organic semiconductor device may be shielded by a resin.

The organic semiconductor material according to the present invention has a symmetric cyclic strucrture in which the molecule has a conjugated electron bonding system and which includes conjugated rings such as benzene rings or thiophene rings and a ethylene chain connecting the rings. In the organic semiconductor material according to the present invention, when the material is composed of a benzene ring, the number of π electrons is basically a multiple of 8, while when the material is composed of a thiophene ring, the number of π electrons is basically a multiple of 4, and a total number can be expressed by 4L (wherein L is 0 or a positive integer). In order to realize a number of n electrons of 4L ± 2 which stabilizes the conjugate system by aromatization, the material is chacterized by beign easily oxidied or reduced in units of two π electrons. In other words, the material has an oxidation or reduction mechanism (i.e., electrons are emitted or donated) in units of two n electrons. In addition, a two- or three-dimensional conduction path is formed, and concequently high conductivity can be stably obtained. Further, the organic semiconductor material according to the present invention can be dissolved in a large variety of organic solvents at room temperature and thus can be used for forming films at room temperature based on various coating methods. Therefore, a high-mobility semiconductor device can be manufactured using, for exmaple, a coating method such as a spin coating method or an ink jet printing method. As a result, for example, a large-area TFT array can be manufactured at low cost using a simple apparatus.

### [Brief Description of the Drawings]

[Fig. 1] Fig. 1 is a drawing alternative to a photograph of single crystal X-ray structure analysis of (2,2,2,2)-paracyclophanetetraene.
[Fig. 2] Fig. 2 is a drawing alternative to a photograph of single crystal X-ray structure analysis of 2,5-thiophenophanetetraene.
[Fig. 3] Fig. 3(A) is a diagram showing a LUMO band dispersion calculated for (2,2,2,2)-paracyclophanetetraene on the basis of its crystal structure, and Fig. 3(B) is a diagram showing a HOMO band dispersion calculated for 2,5-thiophenophanetetraene on the basis of its crystal structure.
[Fig. 4] Fig. 4(A) is a graph showing the results of measurement of two-terminal voltage-current characteristics of a (2,2,2,2)-paracyclophanetetraene single crystal in Example 1, and Fig. 4(B) is a graph showing a relation (I-V characteristics) between gate voltage and drain current of a test product of an organic field effect transistor made on an experimental basis in Example 2.
[Fig. 5] Fig. 5(A) is a schematic partial sectional view of a bottom gate/top contact-type organic field effect transistor, and Fig. 5(B) is a schematic partial sectional view of a bottom gate/bottom contact-type organic field effect transistor.
[Fig. 6] Fig. 6(A) is a schematic partial sectional view of a top gate/top contact-type organic field effect transistor, and Fig. 6(B) is a schematic partial sectional view of a top gate/bottom contact-type organic field effect transistor.
[Fig. 7] Fig. 7 is a schematic partial sectional view of a test product of an organic field effect transistor in Example 2.

### [Best Mode for Carrying Out the Invention]

The present invention will be described on the basis of embodiments with reference to the drawings.

### (Example 1)

Example 1 relates to an organic semiconductor device according to a first or second embodiment of the present invention and to an organic semiconductor thin film according to the first embodiment of the present invention. The organic semiconductor device of Example 1 includes a channel forming region including an organic semiconductor thin film which is composed of an organic semiconductor material having a oxidation or reduction mechanism in units of two π electrons and a two- or three-dimensional conduction path. Alternatively, the organic semiconductor device includes a channel forming region including an organic semiconductor thin film which is composed of an organic semiconductor material having the following general formula (1) or (1') (wherein a hydrogen atom constituting a benzene ring may be substituted, and n is 0 or a positive integer).

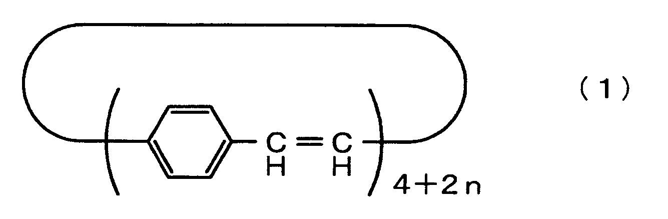

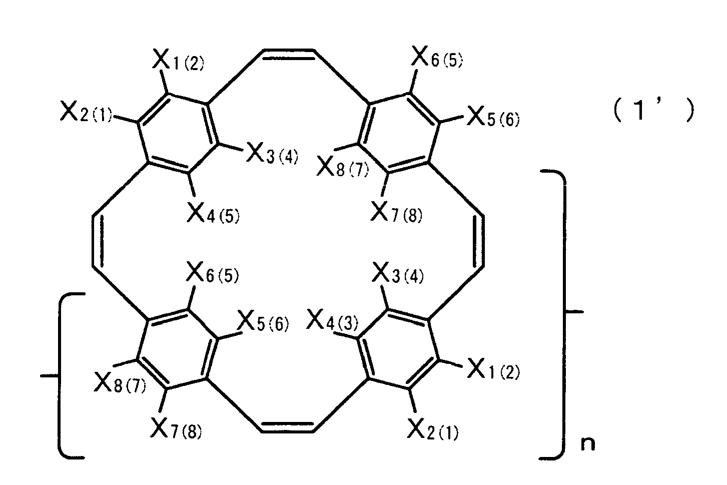

In general formula (1') or general formula (2') which will be described below, X₁, X₂, X₃, X₄, X₅, X₆, X₇, and X₈ each represent a hydrogen atom, an alkyl group (CₘH₂ₘ₊₁- wherein m = 1, 2, 3, ...), or a halogen atom (specifically, a F atom, a Cl atom, a Br atom, or a I atom); the notations X₁₍₂₎ and X₂₍₁₎ represent that when X₁ and X₂ are not the same atom or alkyl group, an organic semiconductor material in which X₁ is a certain atom or alkyl group (referred to as "α" for the convenience sake) and X₂ is another atom or alkyl group (referred to as "β" for the convenience sake) and an organic semiconductor material in which X₁ is β and X₂ is α can coexist; and this applies to the notations X₃₍₄₎ and X₄₍₃₎, the notations X₅(₆₎ and X₆₍₅₎, and the notations X₇₍₈₎ and X₈₍₇₎. However, in the formula, substituents with the same subscript should be the same from the viewpoint of requirements of a synthesis method.

A general synthetic pathway will be described below. The synthesis can be performed on the basis of Wittig reaction between phosphonium ylide and aldehyde. In the formula, n varies depending on the synthesis conditions and the like.

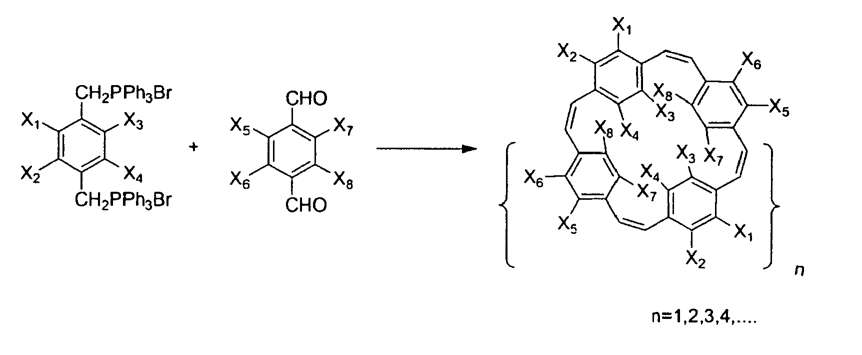

In Example 1, more specifically, (2,2,2,2)-paracyclophanetetraene (abbreviated as "PCT" hereinafter) is synthesized by Wittig reaction described below. Since the reaction product contains analogues including tetramer (n = 0), hexamer (n = 1), octomer (n = 2), and decamer (n = 3), these analogues are separated on the basis of gel permeation chromatography (GPC). As a result of purification, PCT can be obtained in a yield of about 10%. Synthesis of PCT is referred to, for example, Acta Chem. Scand., B 29, (1975), No. 1, pp 138-139, "Simple Synthesis of [2.2.2.2]Paracycrophane-1,9,17,25-tetraene by Wittig Reaction", Bengt Thulin et. al.

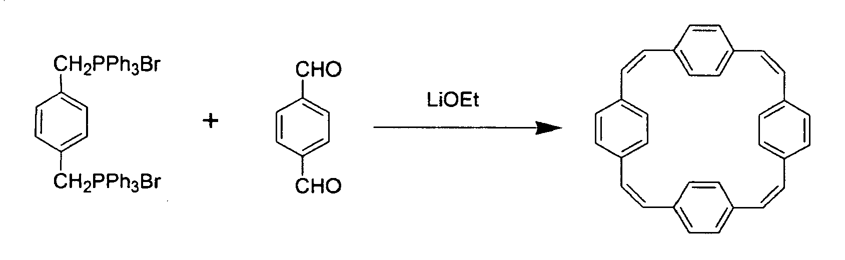

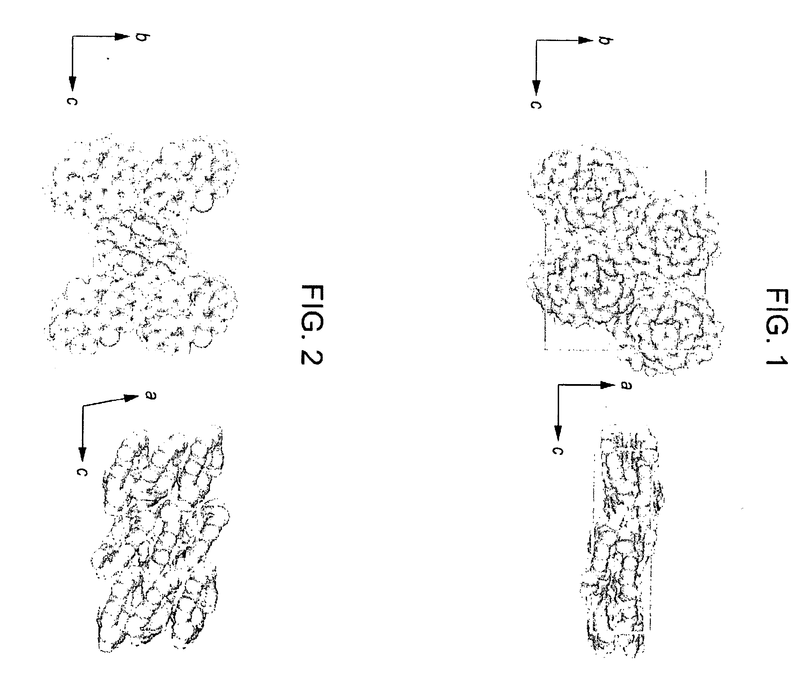

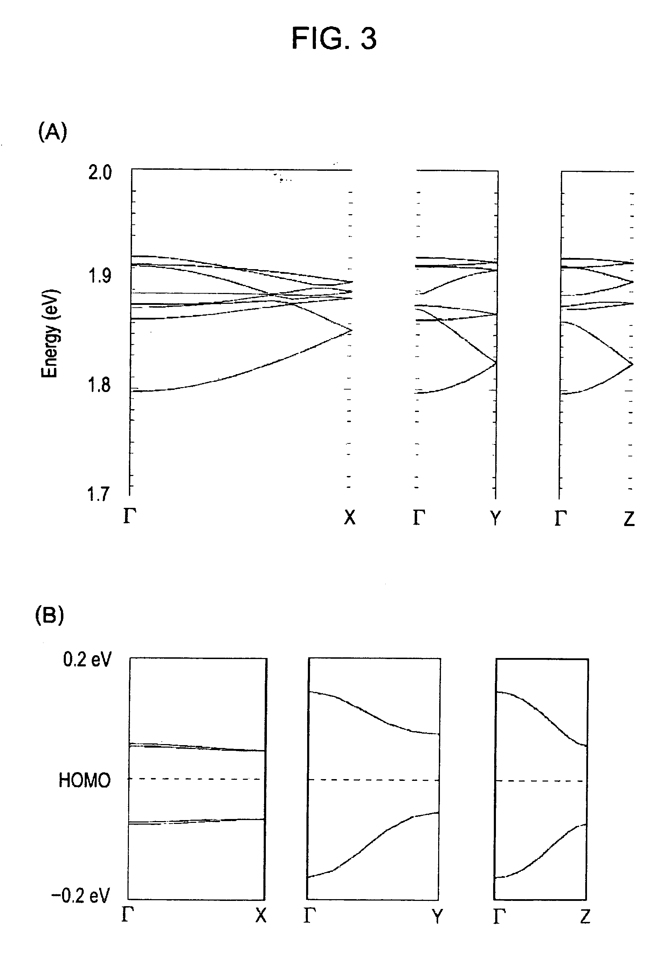

Fig. 1 shows a crystal structure of PCT determined by single crystal X-ray structure analysis. The crystal structure of PCT is known (for example, refer to Acta Cryst., B34, 1889). Also, PCT is expected to exhibit n-type semiconductor characteristics in view of its ionization potential. Therefore, Fig. 3(A) shows the LUMO band dispersion of PCT calculated on the basis of the crystal structure. Since the band disperses in the directions of all reciprocal lattice axes, three-dimensional electron conduction is expected. The three-dimensional conduction path is an important factor for achieving good semiconductor characteristics in view of the fact that a scattering factor is reduced in an organic semiconductor thin film. The band effective mass determined from band dispersion is as low as 1.8 mₑ in the K_{c} axis direction, wherein mₑ is the mass of free electron. The band effective mass has an inversely proportional relation to mobility, and thus a material having small band effective mass can fundamentally become a semiconductor material with high mobility.

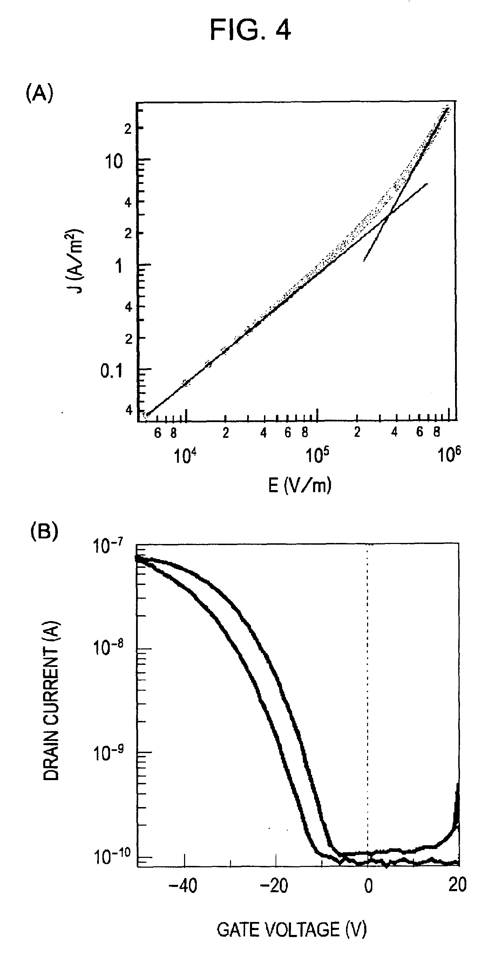

A PCT single crystal formed by sublimation purification and a vapor phase growth method was measured with respect to two-terminal voltage-current characteristics. The results are shown in Fig. 4(A). It was confirmed from Fig. 4(A) that the current value is proportional, not the voltage, but to the square of voltage in a high-electric-field region. This reflects a trap-free conduction mechanism. The mobility calculated from the current density in this region is 1.1 cm²·V⁻¹·sec⁻¹ or more. This result indicates the high possibility that a high-performance thin film device can be obtained using an organic semiconductor device.

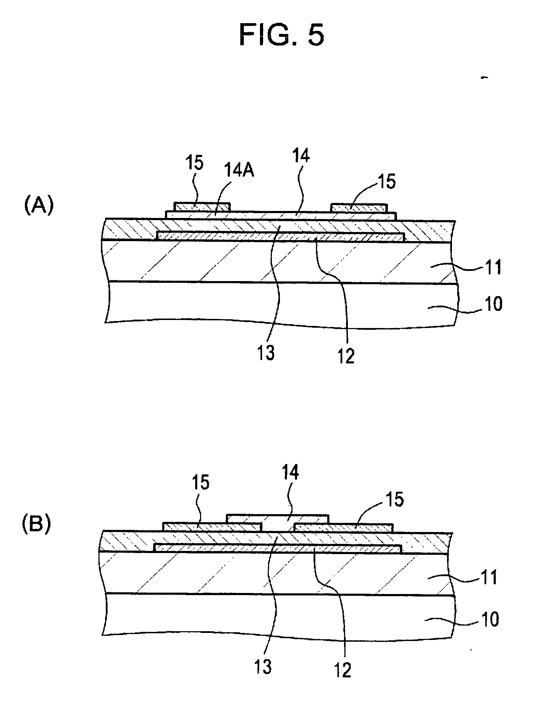

An organic semiconductor device (specifically, an organic field effect transistor) of Example 1 or Example 2 which will be described below includes source/drain electrodes 15, a channel forming region 14 sandwiched between the source/drain electrodes 15, a gate insulating layer 13, and a gate electrode 12 provided opposite to the channel forming region 14 with the gate insulating layer 13 provided therebetween. More specifically, as shown in a schematic partial sectional view of Fig. 5(A), a bottom gate/top contact-type organic field effect transistor of Example 1 or Example 2 described below includes:
(a) a gate electrode 12 formed on substrates 10 and 11 and composed of a gold thin film;
(b) a gate insulating layer 13 formed on the gate electrode 12 and the substrates 10 and 11 and composed of SiO₂;
(c) a channel forming region 14 and channel forming region extensions 14A formed on the gate insulating film 13 and composed of the organic semiconductor thin film of Example 1 or Example 2 described below; and
(d) source/drain electrodes 15 formed on the channel forming region extensions 14A and composed of a gold thin film.
The substrates 10 and 11 include the glass substrate 10 and the insulating film 11 formed on the surface thereof and composed of SiO₂. More specifically, the gate electrode 12 and the gate insulating layer 13 are formed on the insulating film 11.

A method for manufacturing the bottom gate/top contact-type organic field effect transistor (specifically TFT) will be outlined below.

### [Step-100]

First, the gate electrode 12 is formed on the substrate (the glass substrate 10 and the insulating film 11 formed on the surface thereof and composed of SiO₂). Specifically, a resist layer (not shown) in which a portion for forming the gate electrode 12 has been removed is formed on the insulating film 11 on the basis of a lithography technique. Then, a chromium (Cr) layer (not shown) serving as an adhesive layer and a gold (Au) layer as the gate electrode 12 are formed in turn over the entire surface by a vacuum evaporation method, and then the resist layer is removed.
As a result, the gate electrode 12 can be formed on the basis of a so-called liftoff method.

### [step-110]

Next, the gate insulating layer 13 is formed on the substrate (the insulating film 11) including the gate electrode 12. Specifically, the gate insulating layer 13 composed of SiO₂ is formed on the gate electrode 12 and the insulating film 11 on the basis of a sputtering method. In forming the gate insulating layer 13, the gate electrode 12 is partially covered with a hard mask so that a takeoff portion (not shown) of the gate electrode 12 can be formed without using a photolithographic process.

### ] [Step-120]

Next, the channel forming region 14 and the channel forming region extensions 14A are formed on the gate insulating layer 13. Specifically, 10 g of the organic semiconductor material of above-described Example 1 or Example 2 described below is dissolved in 1 L of chloroform to prepare a solution, and the resultant solution is applied on the gate insulating layer 13 by a coating process such as spin coating at room temperature and then dried to form the channel forming region 14 and the channel forming region extensions 14A on the gate insulating layer 13.

### [Step-130]

Then, the source/drain electrodes 15 are formed on the channel forming region extensions 14A so as to hold the channel forming region 14 therebetween. Specifically, a chromium (Cr) layer (not shown) as an adhesive layer and a gold (Au) layer as the source/drain electrodes 15 are formed in turn over the entire surface on the basis of the vacuum evaporation process. As a result, the structure shown in Fig. 5(A) can be obtained. In forming the source/drain electrodes 15, the channel forming region extensions 14A are partially covered with a hard mask so that the source/drain electrodes 15 can be formed without using a photolithographic process.

### [Step-140]

Finally, an insulating layer (not shown) serving as a passivation film is formed over the entire surface, and apertures are formed in the insulating layer above the source/drain electrodes 15. Then, a wiring material layer is formed over the entire surface including the insides of the apertures and then patterned to form wiring (not shown) connected to the source/drain electrodes 15 on the insulating layer, thereby producing a bottom gate/top contact-type organic field effect transistor.

The organic field effect transistor is not limited to the bottom gate/top contact type shown in Fig. 5(A), and may be another type such as a bottom gate/bottom contact type, a top gate/top contact type, or a top gate/bottom contact type.

A bottom gate/bottom contact-type organic field effect transistor shown in a schematic partial sectional view of Fig. 5(B) includes:
(a) a gate electrode 12 formed on substrates 10 and 11;
(b) a gate insulating layer 13 formed on the gate electrode 12 and the substrates 10 and 11;
(c) source/drain electrodes 15 formed on the gate insulating layer 13; and
(d) a channel forming region 14 formed between the source/drain electrodes 15 on the gate insulating layer 13.

A method for manufacturing a bottom gate/bottom contact type TFT will be outlined.

### [Step-200]

First, like in step-100, the gate electrode 12 is formed on the base (the insulating film 11). Then, like in step-110, the gate insulating layer 13 is formed on the gate electrode 12 and the insulating film 11.

### [Step-210]

Next, the source/drain electrodes 15 composed of a gold (Au) layer are formed on the gate insulating layer 13. Specifically, a resist layer in which portions for forming the source/drain electrodes 15 have been removed is formed on the gate insulating layer 13 on the basis of a lithographic technique. Then, like in step-100, a chromium (Cr) layer (not shown) serving as an adhesive layer and a gold (Au) layer as the source/drain electrodes 15 are formed in turn on the resist layer and the gate insulating layer 13 by a vacuum evaporation method. Then, the resist layer is removed. As a result, the source/drain electrodes 15 ca be formed on the basis of a liftoff method.

### [Step-220]

Then, the channel forming region 14 is formed between the source/drain electrodes 15 on the gate insulating layer 13 on the basis of the same method as in step-120. As a result, the structure shown in Fig. 5(B) can be formed.

### [Step-230]

Finally, the same step as step-140 is performed to produce a bottom gate/bottom contact type organic field effect transistor.

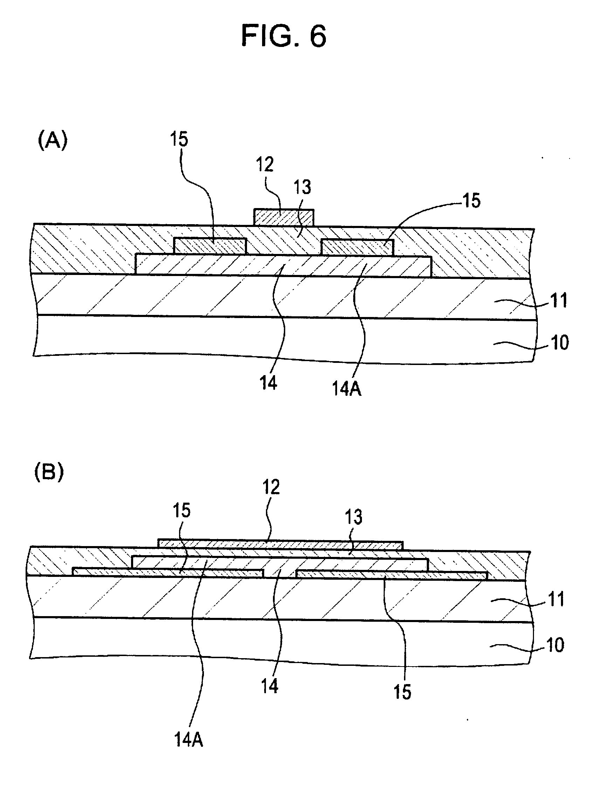

A top gate/top contact-type organic field effect transistor shown in a schematic partial sectional view of Fig. 6(A) includes:
(a) a channel forming region 14 and channel forming region extensions 14A formed on substrates 10 and 11;
(b) source/drain electrodes 15 formed on the channel forming region extensions 14A;
(c) a gate insulating layer 13 formed on the source/drain electrodes 15 and the channel forming region 14; and
(d) a gate electrode 12 formed on the gate insulating layer 13.

A method for manufacturing a top gate/top contact type TFT will be outlined.

### [Step-300]

First, the channel forming region 14 and the channel forming region extensions 14A are formed on the substrate (the glass substrate 10 and the insulating film 11 formed on the surface thereof and composed of SiO₂) on the basis of the same method as in step-120.

### [Step-310]

Then, the source/drain electrodes 15 are formed on the channel forming region extensions 14A so as to hold the channel forming region 14 therebetween. Specifically, a chromium (Cr) layer (not shown) as an adhesive layer and a gold (Au) layer as the source/drain electrodes 15 are formed in turn over the entire surface on the basis of the vacuum evaporation process. In forming the source/drain electrodes 15, the channel forming region extensions 14A are partially covered with a hard mask so that the source/drain electrodes 15 can be formed without using a photolithographic process.

### [Step-320]

Next, the gate insulating layer 13 is formed on the source/drain electrodes 15 and the channel forming region 14. Specifically, the gate insulating layer 13 can be formed by the spin coating method of depositing PVA over the entire surface.

### [Step-330]

Then, the gate electrode 12 is formed on the gate insulating layer 13. Specifically, a chromium (Cr) layer (not shown) as an adhesive layer and a gold (Au) layer as the gate electrode 12 are formed in turn over the entire surface on the basis of the vacuum evaporation process. As a result, the structure shown in Fig. 6(A) can be obtained. In forming the gate electrode 12, the gate insulating layer 13 is partially covered with a hard mask so that the gate electrode 12 can be formed without using a photolithographic process. Finally, the same step as step-140 is performed to produce a top gate/top contact-type organic field effect transistor.

A top gate/bottom contact-type organic field effect transistor shown in a schematic partial sectional view of Fig. 6(B) includes:
(a) source/drain electrodes 15 formed on substrates 10 and 11;
(b) a channel forming region 14 formed between the source/drain electrodes 15 on the substrates 10 and 11;
(c) a gate insulating layer 13 formed on the channel forming region 14; and
(d) a gate electrode 12 formed on the gate insulating layer 13.

A method for manufacturing a top gate/bottom contact type TFT will be outlined.

### [Step-400]

First, the source/drain electrodes 15 are formed on the substrate (the glass substrate 10 and the insulating film 11 formed on the surface thereof and composed of SiO₂). Specifically, a chromium (Cr) layer (not shown) as an adhesive layer and a gold (Au) layer as the source/drain electrodes 15 are formed in turn on the insulating film 11 on the basis of the vacuum evaporation process. In forming the source/drain electrodes 15, the substrate (the insulating film 11) is partially covered with a hard mask so that the source/drain electrodes 15 can be formed without using a photolithographic process.

### [Step-410]

Then, the channel forming region 14 is formed between the source/drain electrodes 15 on the substrate (the insulating film 11) on the basis of the same method as in step-120. Actually, the channel forming region extensions 14A are formed on the source/drain electrodes 15.

### [Step-420]

Next, the gate insulating layer 13 is formed on the source/drain electrodes 15 and the channel forming region 14 (actually on the channel forming region 14 and the channel forming region extensions 14A) by the same method as in step-320.

### [Step-430]

Then, the gate electrode 12 is formed on the gate insulating layer 13 by the same method as in step-330. As a result, the structure shown in Fig. 6(B) can be obtained. Finally, the same step as step-140 is performed to produce a top gate/bottom contact-type organic field effect transistor.

As described above, in Example 2 described below, the organic semiconductor device may be of any one of the bottom gate/top contact type, the bottom gate/bottom contact type, the top gate/top contact type, and the top gate/bottom contact type, and can be manufactured on the basis of the above-described method.

Further, the organic semiconductor material of Example 1 or Example 2 described below is prepared (concentration: 10 g/l) at room temperature using as a solvent each of ethyl acetate, acetone, toluene, tetrahydrofuran, tetrahydropyran, cyclopentanone, and mesitylene instead of chloroform. Further, a test product of an organic field effect transistor is made on an experimental basis by the same method using each of the prepared solutions and then the operation thereof is confirmed. As a result, an organic semiconductor thin film can be formed using any one of the prepared solutions. Further, gate modulation can be confirmed, and it can be confirmed that any one of the organic semiconductor thin films functions as a channel forming region.

### [Example 2]

Example 2 relates to an organic semiconductor device according to the first or third embodiment of the present invention and to an organic semiconductor thin film according to the second embodiment of the present invention. The organic semiconductor device of Example 2 includes a channel forming region including an organic semiconductor thin film which is composed of an organic semiconductor material having an oxidation or reduction mechanism in units of two π electrons and a two- or three-dimensional conduction path. Alternatively, the organic semiconductor device includes a channel forming region including an organic semiconductor thin film which is composed of an organic semiconductor material having the following general formula (2) or (2') (wherein a hydrogen atom constituting a thiophene ring may be substituted, and n is 0 or a positive integer).

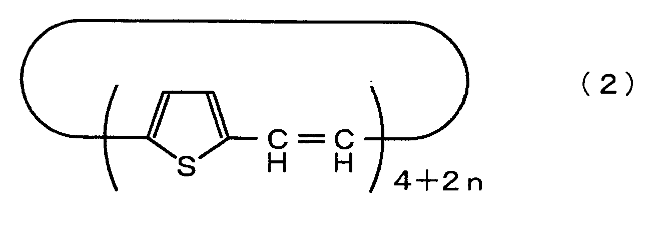

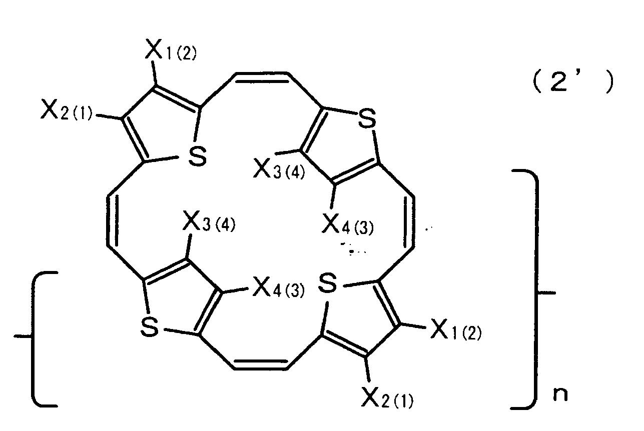

A general synthetic pathway will be described below. The synthesis can be performed on the basis of Wittig reaction between phosphonium ylide and aldehyde. In the formula, n varies depending on the synthesis conditions and the like.

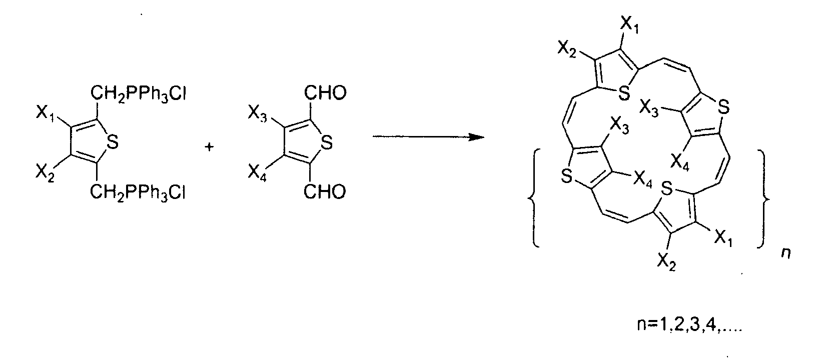

In Example 2, more specifically, 2,5-thiophenophanetetraene (abbreviated as "25TT" hereinafter) is synthesized by Wittig reaction described below. A corresponding phosphonium salt can be synthesized on the basis of the path described below using thiophene as a starting material. Like in Example 1, the by-products other than tetramer (n = 0) are separated from the analogues to obtain 25TT in a yield of about 7%. Synthesis of 25TT is referred to, for example, Acta Chem. Scand., B31, (1977), No. 6, pp. 521-523, "Synthesis of [24] (2,5)-Thiophenophanetetraene or [24] Annulene Tetrasulfide", Anders Strand et. al.

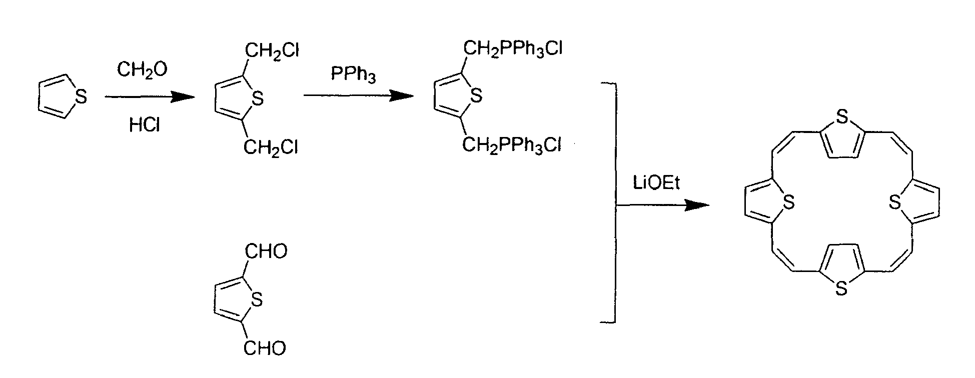

Fig. 2 shows a crystal structure of 25TT determined by single crystal X-ray structure analysis. The crystallographic data is as shown in Table 1 below. Since 25TT is expected to exhibit p-type semiconductor characteristics, Fig. 3(B) shows the HOMO band dispersion of 25TT calculated on the basis of the crystal structure. Since the band disperses along the k_{b} axis and k_{c} axis, two-dimensional electron conduction is expected. The two-dimensional conduction path is also an important factor for achieving good semiconductor characteristics in view of the fact that a scattering factor is reduced in an organic semiconductor thin film. The band effective mass determined from band dispersion is as low as about 1.5 mₑ near point Γ in the K_{c} axis direction, wherein mₑ is the mass of free electron. This value is smaller than that (1.6 mₑ) of pentacene which is known as an excellent semiconductor material, and thus excellent conduction characteristics can be expected.

**[Table 1]**

| |
|---|
| Monoclinic system |
| Space group: P2_{1/n} |
| a = 9.6432(6) Å |
| b = 12.1612(7) Å |
| c = 17.5978(11) Å |
| β 95.795 (2) ° |
| V = 2053.2(2) Å³ |
| Z = 4 |
| R/R_{w} = 0.0334/ (0.0939) |

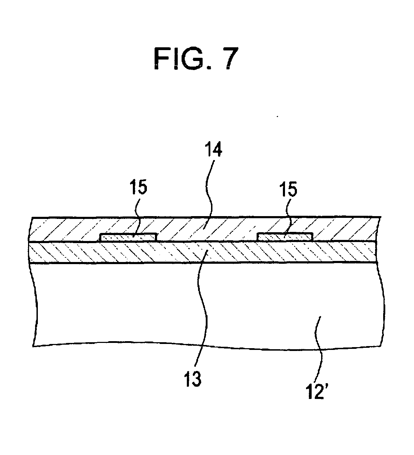

A test product of an organic field effect transistor (refer to a schematic partial sectional view of Fig. 7) including a channel forming region formed on the basis of the coating process such as spin coating at room temperature using a chloroform solution (concentration: 10 g/l) of 25TT, and the operation of the product was confirmed. Specifically, a gate insulating layer 13 is formed by oxidizing the surface of a highly doped silicon semiconductor substrate 12' (functioning as a gate electrode). Then, a gold thin film was deposited to a thickness of 50 nm by evaporation to form source/drain electrodes 15 (length 15 µm). Then, a channel forming region 14 including an organic semiconductor thin film was formed by the spin coating method at room temperature using the 25TT chloroform solution (concentration: 10 g/l). The distance (corresponding to a gate length) between the source/drain electrodes 15 was 5 µm. As a result, as shown in Fig. 4(B), gate modulation could be confirmed, and it could be confirmed that the organic semiconductor thin film having p-type conductivity functions as the channel forming region 14. A mobility of 1 × 10⁻⁵ cm²·V⁻¹·sec⁻¹ could be achieved in a saturation region depending on the spin coating conditions, and the on/off ratio was about 10³.

Although the present invention is described on the basis of the preferred examples, the present invention is not limited to these examples. The structures, constitutions, manufacturing conditions, and manufacturing methods of organic semiconductor devices are just examples and can be appropriately changed. When the organic semiconductor device produced according to the present invention is applied to and used with display devices and various kinds of electronic devices, the organic semiconductor device may be formed as a monolithic integrated circuit in which a large number of organic semiconductor devices are integrated on a substrate or a support. Each organic semiconductor device can be cut and separately used as a discrete component.

## Claims

1. An organic semiconductor device comprising a channel forming region including an organic semiconductor thin film which is composed of an organic semiconductor material having the following general formula (1), wherein some or all of the hydrogen atoms of the benzene ring may be substituted, and n is 0 or a positive integer:

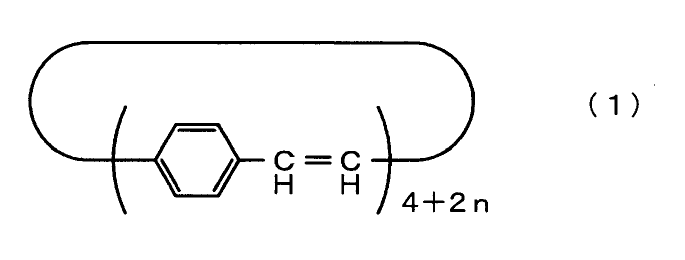

2. The organic semiconductor device according to claim 1, wherein a substituent is an alkyl group or a halogen atom.

3. An organic semiconductor device comprising a channel forming region including an organic semiconductor thin film which is composed of an organic semiconductor material having the following general formula (2), wherein some or all of the hydrogen atoms of the thiophene ring may be substituted, and n is 0 or a positive integer:

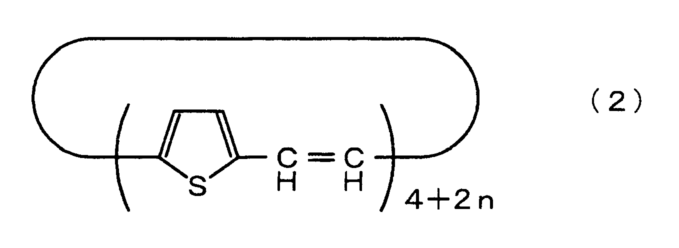

4. The organic semiconductor device according to claim 3, wherein a substituent is an alkyl group or a halogen atom.

5. An organic semiconductor thin film comprising an organic semiconductor material having the general formula (1) below, wherein some or all of the hydrogen atoms of the benzene ring may be substituted, and n is 0 or a positive integer:

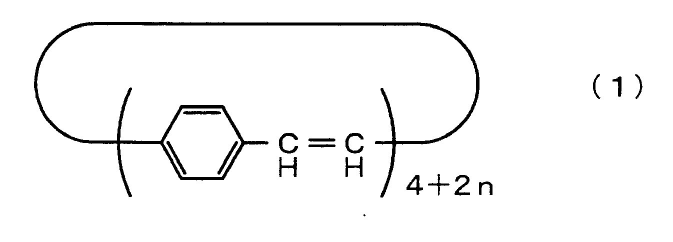

6. The organic semiconductor thin film according to claim 5, wherein a substituent is an alkyl group or a halogen atom.

7. An organic semiconductor thin film comprising an organic semiconductor material having the general formula (2) below, wherein some or all of the hydrogen atoms of the thiophene ring may be substituted, and n is 0 or a positive integer:

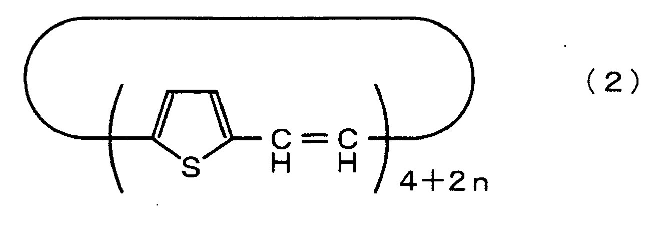

8. The organic semiconductor thin film according to claim 7, wherein a substituent is an alkyl group or a halogen atom.

## Patentansprüche

1. Organisches Halbleiterelement umfassend einen kanalbildenden Bereich, welcher einen organischen Halbleiterdünnfilm beinhaltet, welcher aus einem organischen Halbleitermaterial zusammengesetzt ist, welches die folgende allgemeine Formel (1) aufweist, wobei manche oder alle der Wasserstoffatome des Benzolrings substituiert sein können und n 0 oder eine positive ganze Zahl ist:

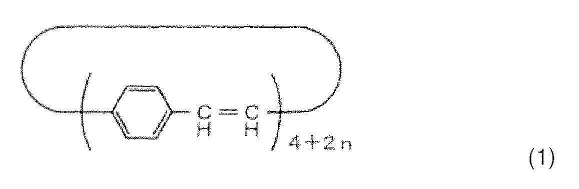

2. Organisches Halbleiterelement nach Anspruch 1, wobei der Substituent eine Alkylgruppe oder ein Halogenatom ist.

3. Organisches Halbleiterelement umfassend einen kanalbildenden Bereich, welcher einen organischen Halbleiterdünnfilm beinhaltet, welcher aus einem organischen Halbleitermaterial zusammengesetzt ist, welches die folgende allgemeine Formel (2) aufweist, wobei manche oder alle der Wasserstoffatome des Thiophenrings substituiert sein können und n 0 oder eine positive ganze Zahl ist:

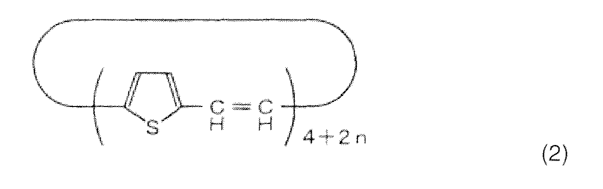

4. Organisches Halbleiterelement nach Anspruch 3, wobei der Substituent eine Alkylgruppe oder ein Halogenatom ist.

5. Organischer Halbleiterdünnfilm umfassend ein organisches Halbleitermaterial, welches die nachstehende allgemeine Formel (1) aufweist, wobei manche oder alle der Wasserstoffatome des Benzolrings substituiert sein können und n 0 oder eine positive ganze Zahl ist:

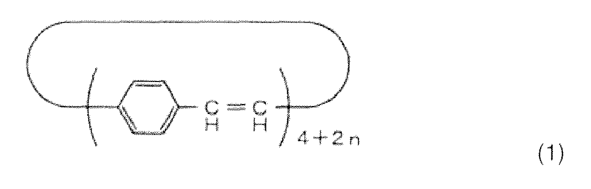

6. Organischer Halbleiterdünnfilm nach Anspruch 5, wobei der Substituent eine Alkylgruppe oder ein Halogenatom ist.

7. Organischer Halbleiterdünnfilm umfassend ein organisches Halbleitermaterial, welches die nachstehende allgemeine Formel (2) aufweist, wobei manche oder alle der Wasserstoffatome des Thiophenrings substituiert sein können und n 0 oder eine positive ganze Zahl ist:

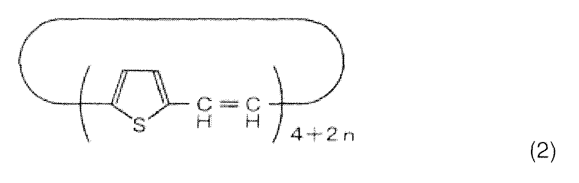

8. Organischer Halbleiterdünnfilm nach Anspruch 7, wobei der Substituent eine Alkylgruppe oder ein Halogenatom ist.

## Revendications

1. Dispositif semi-conducteur organique comprenant une région formant un canal incluant un mince film semi-conducteur organique qui est constitué d'un matériau semi-conducteur organique ayant la formule générale (1) suivante, dans laquelle certains ou tous les atomes d'hydrogène du cycle benzène peuvent être substitués, et n est égal à 0 ou à un nombre entier positif :

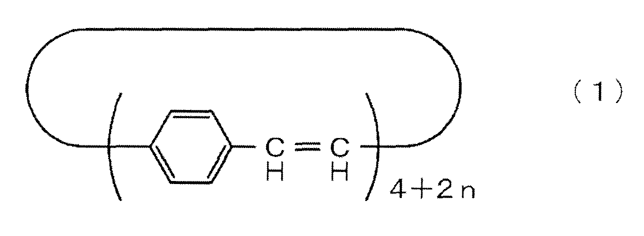

2. Dispositif semi-conducteur organique selon la revendication 1, dans lequel un substituant est un groupe alkyle ou un atome d'halogène.

3. Dispositif semi-conducteur organique comprenant une région formant un canal incluant un mince film semi-conducteur organique qui est constitué d'un matériau semi-conducteur organique ayant la formule générale (2) suivante, dans laquelle certains ou tous les atomes d'hydrogène du cycle thiophène peuvent être substitués, et n est égal à 0 ou à un nombre entier positif :

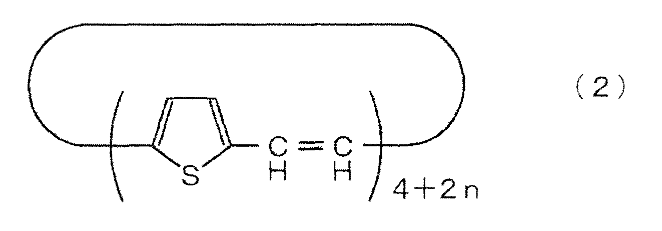

4. Dispositif semi-conducteur organique selon la revendication 3, dans lequel un substituant est un groupe alkyle ou un atome d'halogène.

5. Mince film semi-conducteur organique comprenant un matériau semi-conducteur organique ayant la formule générale (1) ci-dessous, dans laquelle certains ou tous les atomes d'hydrogène du cycle benzène peuvent être substitués, et n est égal à 0 ou à un nombre entier positif :

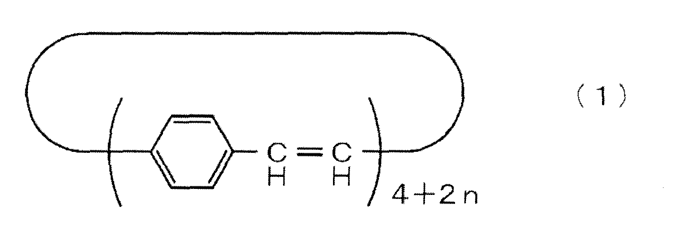

6. Mince film semi-conducteur organique selon la revendication 5, dans lequel un substituant est un groupe alkyle ou un atome d'halogène.

7. Mince film semi-conducteur organique comprenant un matériau semi-conducteur organique ayant la formule générale (2) ci-dessous, dans laquelle certains ou tous les atomes d'hydrogène du cycle thiophène peuvent être substitués, et n est égal à 0 ou à un nombre entier positif :

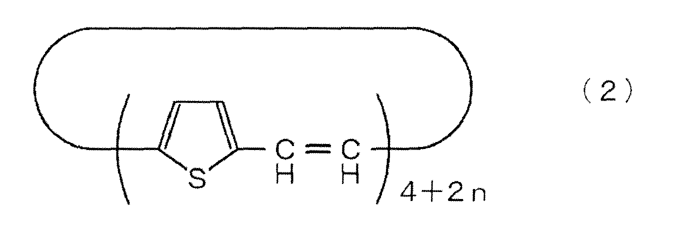

8. Mince film semi-conducteur organique selon la revendication 7, dans lequel un substituant est un groupe alkyle ou un atome d'halogène.
